# EUROPEAN PATENT APPLICATION

(11) **EP 4 810 053 A1**
(43) Date of publication of application: **23.09.2026**
(21) Application number: 23958637.3
(22) Date of filing: 17.11.2023
(51) Int. Cl.: A61K 38/05, A61K 31/4172, A61P 13/12, A61P 19/06

(54) **IMIDAZOLE DIPEPTIDE COMPOSITION WITH RENAL PROTECTIVE EFFECT AND USE THEREOF**

(71) Applicant: Shanghai Lytone Biochemicals, Ltd., Shanghai 200050 (CN); Lytone Enterprise, Inc., New Taipei City 221 (TW)
(72) Inventor: KONG, Xiangru, Shanghai 200050 (CN); TSENG, Weiting, Shanghai 200050 (CN); CHANG, Weiting, Shanghai 200050 (CN); CHANG, William T.H., Shanghai 200050 (CN)
(74) Representative: Barker Brettell LLP
(86) International application number: PCT/CN2023/132357
(87) International publication number: WO 2025/102364

(57) **Abstract**

Disclosed are an imidazole dipeptide composition with a renal protective effect and use thereof. The imidazole dipeptide composition with a renal protective effect comprises anserine and carnosine, wherein, on the basis of the total weight of anserine and carnosine being 100%, the content of anserine is 10%-99% and the content of carnosine is 1%-90%. Mouse experiments confirm that mice with hyperuricemic renal injury, after continuously receiving the imidazole dipeptide composition for a period of time, show significantly less renal injury compared to the control group, which indicates that the imidazole dipeptide composition has an excellent renal protective effect.

## Description

### TECHNICAL FIELD

The present disclosure relates to an imidazole dipeptide composition and the use thereof, and specifically to an imidazole dipeptide composition with a renal protective effect and the use thereof in the manufacturing of related products with a renal protective effect, such as medicaments or foods for special medical purposes (FSMP).

### BACKGROUND ART

The kidneys are vital organs in the human body, and mainly function to eliminate metabolites and certain wastes and toxic substances from the body by producing urine, while retaining water and other useful substances via the reabsorption function. Poor dietary habits may lead to an elevated uric acid level. Persistent hyperuricemia may induce a variety of complications such as gout, diabetes, metabolic syndrome, oxidative stress, inflammation, hypertension and endothelial dysfunction, and may also impose a heavy burden on the kidneys and induce kidney injury.

Creatinine and urea nitrogen are common biochemical indicators of renal function. Creatinine is a waste product produced by human muscles. Since almost all creatinine is excreted by the kidneys and rarely affected by diet, it serves as a relatively objective indicator of renal function. An increase in the blood creatinine concentration indicates that the kidneys' ability to eliminate waste products is impaired. Furthermore, urea nitrogen is converted in the liver via the urea cycle from a metabolite produced by degradation of proteins during digestion. If the renal function is impaired, the ability to excrete urea decreases accordingly, resulting in urea accumulation in the blood and an elevated blood urea nitrogen level.

CN 113616688 A discloses a composition for repairing renal injury and the use thereof. The composition comprises active components consisting of anserine (β-alanyl-1-methyl-L-histidine, Ans) powder, Radix Astragali extract, and Sophorae flavescentis radix extract. The mass ratio of the anserine powder, Radix Astragali extract, and Sophorae flavescentis radix extract in the composition is 80-90 : 3-10 : 5-15. Relevant experiments demonstrate that the composition consisting of the anserine powder, Radix Astragali extract, and Sophorae flavescentis radix extract has a certain effect in repairing renal injury, while anserine alone delivers unsatisfactory efficacy in renal injury repair. In addition, the Radix Astragali extract and Sophorae flavescentis radix extract used in the composition have an unpleasant taste characteristic of traditional Chinese medicines.

### SUMMARY OF THE INVENTION

An objective of the present disclosure is to provide a composition with a renal protective effect.

Another objective of the present disclosure is to provide the use of the composition.

The present disclosure provides the following technical solutions.

Technical solution 1: An imidazole dipeptide composition with a renal protective effect, comprising anserine and carnosine, wherein based on 100% of the total weight of the anserine and the carnosine, the content of the anserine is 10%-99% and the content of carnosine is 1%-90%.

Technical solution 2: The imidazole dipeptide composition according to technical solution 1, wherein based on 100% of the total weight of the anserine and the carnosine, the content of the anserine is 91% and the content of the carnosine is 9%.

According to some specific embodiments of the present disclosure, in the imidazole dipeptide composition of the present disclosure, the weight sum of anserine and carnosine accounts for 50% or more of the total weight of the composition, preferably 60% or more, further preferably 70% or more, 80% or more, 85% or more, 90% or more, 95% or more or 100%.

Technical solution 3: The imidazole dipeptide composition according to technical solution 1 or 2, consisting of anserine and carnosine.

Technical solution 4: The imidazole dipeptide composition according to technical solution 1 or 2, further comprising other substances with a renal protective effect. According to some specific embodiments of the present disclosure, the amount of the other substances with a renal protective effect accounts for 50% or less of the total weight of the imidazole dipeptide composition of the present disclosure.

Technical solution 5: The imidazole dipeptide composition according to technical solution 4, wherein the other substances with a renal protective effect include histidine and/or a histidine derivative. The histidine derivative includes, but is not limited to: oligopeptides containing histidine residues, that is, oligopeptides consisting of a plurality of histidine residues, or consisting of histidine and other amino acids. In particular, they may be dipeptides, tripeptides, etc., preferably dipeptides. The histidine and the histidine derivative may be derived from animals and/or plants, such as from chicken, fish or other animal muscles.

Technical solution 6: Use of the imidazole dipeptide composition according to any one of technical solutions 1-5 in the manufacturing of a composition with a renal protective effect.

In the present disclosure, the term "renal protective" means having a protective effect on the kidneys, including assisting in the protection of the kidneys and benefiting kidney health.

According to some specific embodiments of the present disclosure, the renal protective effects include alleviating renal injury caused by hyperuricemia.

According to some specific embodiments of the present disclosure, the renal protective effects include reducing uric acid levels, serum creatinine and/or urea nitrogen levels in individuals with renal injury caused by hyperuricemia.

According to some specific embodiments of the present disclosure, the renal protective effects include ameliorating renal tissue injury caused by hyperuricemia and alleviating inflammatory cell infiltration in renal tissues.

According to some specific embodiments of the present disclosure, the renal protective effects include reducing the levels of pro-inflammatory factors including TNF-α, IL-1β and IL-6 in individuals with renal injury.

According to some specific embodiments of the present disclosure, the renal protective effects include increasing glutathione levels in the serum and/or renal tissues of individuals with hyperuricemia.

According to some specific embodiments of the present disclosure, the renal protective effects include increasing SOD levels in the serum and/or renal tissues of individuals with hyperuricemia and alleviating oxidative damage.

According to some specific embodiments of the present disclosure, the renal protective effects include reducing malondialdehyde levels in the serum and/or renal tissues of individuals with hyperuricemia.

The present disclosure also provides a method for protecting the kidneys, the method comprising administering to an individual in need thereof an effective amount of the imidazole dipeptide composition according to any one of technical solutions 1-5 of the present disclosure.

According to some specific embodiments of the present disclosure, the individual is a mammal or a human.

Technical solution 7: The use according to technical solution 6, wherein the composition is for use in manufacturing a medicament.

Technical solution 8: The use according to technical solution 7, wherein the medicament contains an effective amount of the imidazole dipeptide composition.

Technical solution 9: The use according to technical solution 7 or 8, wherein the dosage of the imidazole dipeptide composition in the medicament is 1 mg-200 mg/day, preferably 1 mg-100 mg/day, and more preferably 5 mg-50 mg/day.

Technical solution 10: The use according to any one of technical solutions 7-9, wherein the medicament also contains excipients, preferably, the excipients include one of or a combination of two or more of sorbitol, maltodextrin and magnesium stearate.

Technical solution 11: The use according to any one of technical solutions 7-10, wherein the medicament is in the form of tablets, capsules, powder or granules.

Technical solution 12: The use according to technical solution 6, wherein the composition is for use in manufacturing a food product. The food product may be, for example, a functional food or a dietary supplement. The functional food may be, for example, a health food, foods for special medical purposes (FSMP), and a therapeutic diet.

Technical solution 13: The use according to technical solution 12, wherein the amount of the imidazole dipeptide composition in the foods, based on the consumption of an adult, can be 1 mg-200 mg/day, preferably 1 mg-100 mg/day, and more preferably 5 mg-50 mg/day.

The present disclosure has demonstrated through experiments that the imidazole dipeptide composition of the present disclosure provides excellent protection for the kidneys of mice and effectively alleviates renal injury caused by hyperuricemia.

According to a specific embodiment of the present disclosure, the imidazole dipeptide composition has a good uric acid-lowering effect on mice with renal injury caused by hyperuricemia, and the serum creatinine and urea nitrogen levels in the intervention group decrease significantly to normal levels, indicating that intervention with the imidazole dipeptide can alleviate renal injury caused by hyperuricemia. Histopathological sections of mouse kidneys showed that the imidazole dipeptide composition in the intervention group could significantly ameliorate renal tissue injury caused by hyperuricemia and alleviate inflammatory cell infiltration in renal tissues. Further, the levels of three pro-inflammatory factors, TNF-α, IL-1β, and IL-6, were significantly reduced in the imidazole dipeptide composition intervention group. Still further, the results of glutathione detection in serum and kidney tissue homogenates showed that treatment with the imidazole dipeptide composition could increase glutathione levels in serum and kidney tissue of hyperuricemic model mice and alleviate oxidative damage caused by hyperuricemia. The results of superoxide dismutase detection in serum and kidney tissue homogenates showed that treatment with the imidazole dipeptide composition could increase SOD levels in serum and kidney tissue of hyperuricemic model mice and alleviate oxidative damage caused by hyperuricemia. The results of glutathione peroxidase detection in serum and kidney tissue homogenates showed that treatment with the imidazole dipeptide composition could increase glutathione peroxidase levels in serum and kidney tissue of hyperuricemic model mice and alleviate oxidative damage caused by hyperuricemia. The results of malondialdehyde detection in serum and kidney tissue homogenates showed that treatment with the imidazole dipeptide composition could significantly reduce malondialdehyde levels in serum and kidney tissue of hyperuricemic model mice. The malondialdehyde levels in all intervention groups exhibited no significant difference compared with the normal group, indicating that all treatments could alleviate oxidative damage caused by hyperuricemia.

The imidazole dipeptide composition provided by the present disclosure has a renal protective effect. Mouse experiments confirmed that mice with hyperuricemic renal injury, after continuously receiving the imidazole dipeptide composition of the present disclosure for a period of time, show significantly less renal injury compared to the control group, which indicates that the composition has an excellent renal protective effect.

### BRIEF DESCRIPTION OF THE DRAWINGS

FIG. 1 is a schematic diagram of the animal experiment procedure.
FIG. 2 shows the effects of different interventions on serum uric acid in mice.
FIG. 3 shows the effects of different interventions on serum creatinine levels in mice.
FIG. 4 shows the effects of different interventions on serum urea nitrogen levels in mice.
FIG. 5 shows the pathological analysis of the effects of different interventions on mouse kidneys.
FIG. 6 shows the level of inflammatory factors (TNF-α) in the kidneys of mice.
FIG. 7 shows the level of inflammatory factors (IL-1β) in the kidneys of mice.
FIG. 8 shows the level of inflammatory factors (IL-6) in the kidneys of mice.
FIG. 9 shows the level of inflammatory factors (IL-10) in the kidneys of mice.
FIG. 10 shows serum lipopolysaccharide levels in mice.
FIG. 11 shows serum lactate dehydrogenase levels in mice.
FIG. 12 shows serum glutathione levels in mice.
FIG. 13 shows glutathione levels in renal tissues of mice.
FIG. 14 shows serum superoxide dismutase levels in mice.
FIG. 15 shows superoxide dismutase levels in renal tissues of mice.
FIG. 16 shows serum glutathione peroxidase levels in mice.
FIG. 17 shows glutathione peroxidase levels in renal tissues of mice.
FIG. 18 shows serum malondialdehyde levels in mice.
FIG. 19 shows malondialdehyde levels in renal tissues of mice.

Data are presented as mean ± standard deviation in the figures. Letters a, b, c and the like indicate the results of significance analysis for data in the same column (p < 0.05). Values marked with the same letter show no significant difference.

### DETAILED DESCRIPTION OF EMBODIMENTS

In order to understand the technical features, objectives and beneficial effects of the present disclosure more clearly, the technical solutions of the present disclosure are described in detail as below, but cannot be construed as limitations on the implementable scope of the present disclosure.

### Example 1

This example provides an imidazole dipeptide composition. The composition comprises, in parts by weight:
91 parts of anserine, and
9 parts of carnosine.

Anserine and carnosine were taken in the above ratio and mixed uniformly to obtain the bioactive peptide of this example, which is an imidazole dipeptide composition.

### Example 2

This example provides a medicament comprising the composition provided in Example 1.

### Example 3

This example provides a food comprising the composition provided in Example 1.

### Experiment on the renal protective effect of imidazole dipeptides

The renal protective effect of the imidazole dipeptide of the present disclosure is verified via animal experiments.

### 1. Test samples and test animals

Test samples: The bioactive peptide of Example 1 (i.e., an imidazole dipeptide consisting of 91 parts of anserine and 9 parts of carnosine)

Test animals: Male ICR mice (SPF-grade) aged 5 weeks, purchased from Shanghai JSJ Laboratory Animal Co., Ltd.

### 2. Grouping and treatment of experimental animals

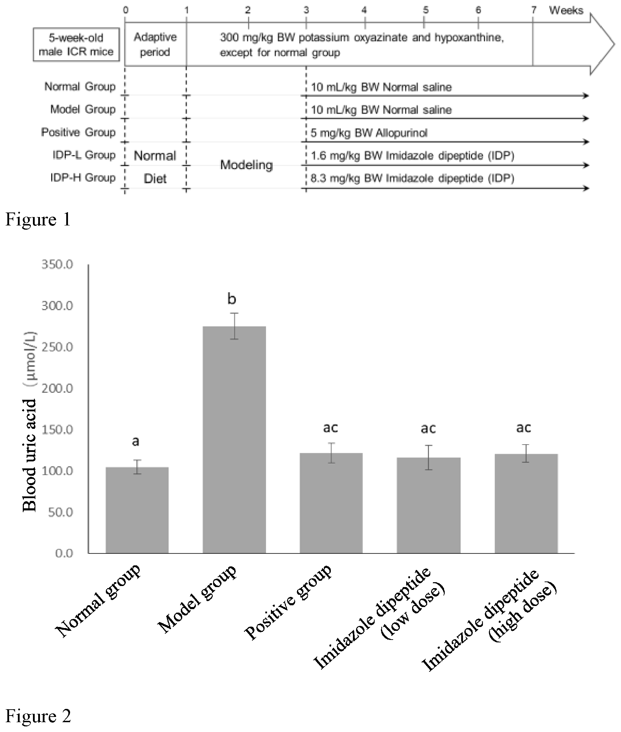

All mice were acclimated under the conditions of a temperature of 24°C ± 2°C, a 12 h light/12 h dark cycle, with free access to food and water. After one week of acclimatization, the mice were randomized, with 7 animals per group, including normal control group, model group, positive control group, low-dose imidazole dipeptide group (IDP-L) and high-dose imidazole dipeptide group (IDP-H). The modeling period was two weeks, and the intervention period was four weeks. During modeling, except for the normal control group, mice in all other groups were intragastrically administered 300 mg/kg potassium oxyazinate and hypoxanthine (suspended in 0.5% CMC-Na solution) daily to establish a hyperuricemia model. During the intervention period, except for the normal group, mice in all other groups were intragastrically administered 300 mg/kg potassium oxyazinate and hypoxanthine daily for persistent modeling. Two hours after modeling, mice in each group were dosed according to the regimens shown in FIG. 1. All test samples were dissolved in normal saline. Mice in the normal group and model group were intragastrically administered an equal dose of normal saline.

### 3. Sampling and treatment of test samples

During modeling, blood was collected via tail amputation once a week to detect changes in serum uric acid. Fresh feces were collected from each group of mice on the last two days of the experiment and stored in a refrigerator at -20°C. Before sacrifice, the mice were fasted for 12 hours and received the final modeling. Blood samples were collected 1 hour after modeling, followed by sacrifice and dissection. Whole blood of mice was kept at 4°C for 2 h, then centrifuged at 3000 rpm for 10 min. The obtained serum was aliquoted and stored in a refrigerator at -80°C. Liver and kidneys of the mice were harvested. One kidney from each mouse was fixed in 4% paraformaldehyde for pathological analysis. All other tissue samples were immediately snap-frozen in liquid nitrogen, aliquoted and stored at -80°C.

### 4. Detection method

The levels of uric acid, creatinine, urea nitrogen, lactate dehydrogenase, xanthine oxidase (XOD), lipopolysaccharide (LPS), oxidative stress indicators (glutathione (GSH), superoxide dismutase (SOD), glutathione peroxidase (GSH-Px) and malondialdehyde (MDA)), inflammatory factors (TNF-α, IL-1β, IL-6 and IL-10), and hypoxanthine phosphoribosyl transferase 1 (HPRT1) were determined in accordance with the methods described in the corresponding kit instructions; the body weight of mice was directly weighed and measured twice a week; the urine pH of mice was tested using pH test strips once a week; renal histopathological analysis was performed in collaboration with Wuhan Servicebio Technology Co., Ltd.; Western blot analysis of renal uric acid transporters was conducted following routine laboratory procedures.

### 5. Data processing method

Statistical analysis was performed using SPSS22.0 software with the Tukey test. A difference of p < 0.05 was considered statistically significant. GraphPadPrism8 and Rstudio were used for plotting, correlation analysis and bioinformatics analysis.

### 6. Experimental results and conclusions

### 6.1 Modeling results

One week after modeling with potassium oxyazinate and hypoxanthine, blood was collected via tail amputation to detect serum uric acid levels. After one week of modeling, serum uric acid levels increased significantly in some of the mice, while no obvious increase was observed in most animals. Therefore, a second round of modeling was conducted for an additional week. After two weeks of modeling, blood was collected again via tail amputation to detect serum uric acid levels of mice. The results showed that after two weeks of modeling, serum uric acid levels in all mice except those in the normal group were significantly elevated and approximately doubled relative to the normal group, indicating that the hyperuricemia model was successfully established. Therefore, starting from the third week, intervention with imidazole dipeptides or probiotics was administered by gavage while modeling continued.

### 6.2 Changes in body weight and urine pH of mice during intragastric administration

Throughout the 6-week intragastric intervention, body weight fluctuated somewhat in some groups, while no significant or obvious changes in body weight were observed in most groups. During the six weeks, urine pH showed irregular fluctuations with small amplitudes, whether compared among different groups at the same time point or within the same group at different time points.

### 6.3 Effects of different interventions on three renal function indicators in mice

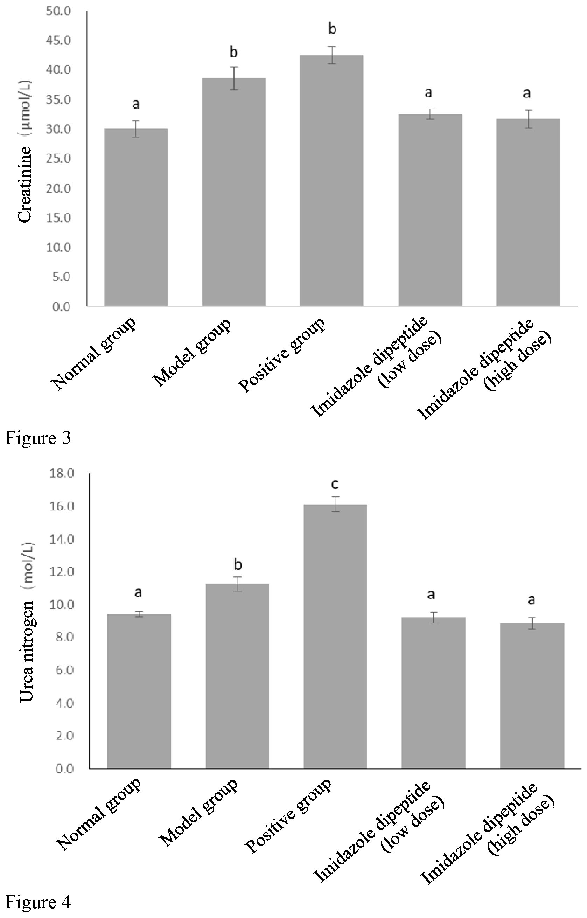

Serum uric acid level is the most critical indicator for direct diagnosis of hyperuricemia. Creatinine is produced via spontaneous and irreversible conversion of creatine phosphate in muscle tissues. Its production remains relatively constant under normal conditions unless there is a dramatic change in muscle mass. The circulating level of free creatinine is entirely determined by its excretion rate. Therefore, determination of creatinine content in serum or plasma can be used to evaluate glomerular filtration efficiency so as to confirm renal function status. Blood urea nitrogen is derived from the liver and excreted via the kidneys into urine. Renal failure, nephritis and urinary tract obstruction can lead to elevated blood urea nitrogen levels. It can be seen that serum creatinine and urea nitrogen are two indicators for evaluating renal function. The detection results of the three renal function indicators are presented in FIG. 2, FIG. 3 and FIG. 4. The model group showed significantly higher uric acid levels than all other groups. After 4 weeks of intervention, uric acid levels in all intervention groups decreased significantly, indicating that interventions with all formulations had good uric acid-lowering effects. The uric acid levels in the positive control group and the low- and high-dose imidazole dipeptide groups decreased to the level of the normal group (no significant difference from the normal group). Uric acid levels in the remaining intervention groups were slightly higher than those in the normal group but significantly lower than those in the model group. Along with elevated uric acid concentration, serum creatinine and urea nitrogen levels in the model group increased significantly compared with the normal group, which is consistent with the results reported in literatures, namely that hyperuricemia is accompanied by renal function damage in mice. Except for the positive control group, serum creatinine and urea nitrogen levels in all other intervention groups decreased significantly to normal levels (with no significant difference from the normal group, but a significant difference from the model group), indicating that intervention with imidazole dipeptide can alleviate renal injury caused by hyperuricemia. The increase in creatinine and urea nitrogen levels in the positive control group of mice indicated that although allopurinol can reduce serum uric acid levels in mice with hyperuricemia, it exhibits strong nephrotoxicity to mice.

### 6.4 Protective effects of different interventions on mouse kidneys

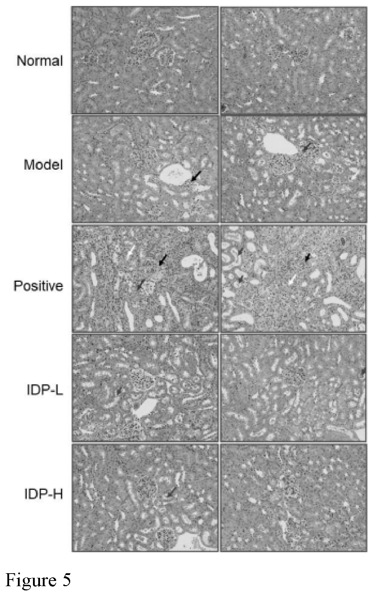

Clinically, changes in renal tissue structure and inflammatory cell infiltration are significant pathological features of hyperuricemia. FIG. 5 shows the histopathological sections of mouse kidneys in each group. As seen under the field of view in the sections from the normal group, the glomerular matrix was uniform, renal tubular epithelial cells were round and plump, and the connective tissue between the urinary tubules was the renal interstitium, with no obvious hyperplasia in the interstitium and no obvious inflammatory changes. In the model group, under the field of view, a small number of lymphocytes infiltrated around blood vessels (black arrows), and granulocytes were present within glomeruli (red arrows). Severe hydropic degeneration was observed in numerous renal tubular epithelial cells, which presented cellular swelling and pale, loose cytoplasm (green arrows). Renal tubules were dilated with flattened epithelial cells and enlarged lumens (blue arrows), which were filled with eosinophilic flocculent materials (purple arrows). A small number of exfoliated epithelioid cells were occasionally found in renal tubules (yellow arrows). In the positive control group, renal tubular atrophy, reduced eosinophilia of epithelial cells, luminal stenosis and blurred tissue structure were visible in the field of view (white arrows), accompanied by mild connective tissue hyperplasia (orange arrows), as well as mild infiltration of lymphocytes (black arrows) and granulocytes (red arrows). Massive hydropic degeneration of renal tubular epithelial cells was observed, which presented cellular swelling and pale, loose cytoplasm (green arrows). Mild renal tubule dilatation, flattened renal tubule epithelial cells and enlarged lumens were occasionally seen, with a few granulocytes present in the lumens (blue arrows). Eosinophilic substances were rare in the lumen of the renal tubules (purple arrows). Compared with the model group, renal tissue damage was significantly alleviated in all other groups. Only mild hydropic degeneration of renal tubular epithelial cells was visible in the field of view, which presented cellular swelling and pale, loose cytoplasm (green arrows). Eosinophilic substances were rare in the lumen of the renal tubules (purple arrows). In the case of hyperuricemia modeling using potassium oxyazinate and hypoxanthine, mice showed obvious inflammation and histological damage in their kidneys. The reabsorption of renal tubules and filtration efficiency of glomeruli exert important influences on uric acid levels; renal tubular injury and glomerular filtration dysfunction can further elevate uric acid concentration and consequently result in the deposition of urate crystals. Although allopurinol can reduce uric acid levels in mice with hyperuricemia, it can cause severe renal injury. Except for the positive control group, all other intervention groups could significantly ameliorate renal tissue injury caused by hyperuricemia and alleviate inflammatory cell infiltration in renal tissues. The pathological scores were determined according to the diagnostic criteria for pathological changes (Table 1), and the results are shown in Table 2.

**Table 1. Pathological scoring criteria**

| Four-point grading system | | |
|---|---|---|
| Score (number) | Type | Description |
| 0 | Within normal range | Under the circumstances of the study, the tissue is considered normal considering the age, sex, strain and other factors of the animals. Changes that occur under other conditions can be considered abnormal. |
| 1 | Minimal | The observed changes marginally exceed the normal range. |
| 2 | Mild | Lesions are detectable but not severe. |
| 3 | Moderate | Lesions are obvious and are quite likely to be more severe. |
| 4 | Severe | Lesions are very serious (lesions have occupied the entire tissue and organ) |

**Table 2. Pathological scores (average score of each group)**

| | Inflammatory cell infiltration | Renal tubular hydropic degeneration | Renal tubular atrophy | Renal tubular dilatation | Connective tissue hyperplasia | Protein casts |
|---|---|---|---|---|---|---|
| Normal group | 0.00 | 0.00 | 0.00 | 0.00 | 0.00 | 0.00 |
| Model group | 0.86 | 1.29 | 0.00 | 0.14 | 0.00 | 0.00 |
| Positive group | 0.71 | 1.14 | 0.29 | 0.57 | 0.29 | 0.14 |
| IDP-L | 0.00 | 1.29 | 0.00 | 0.00 | 0.00 | 0.00 |
| IDP-H | 0.00 | 1.00 | 0.00 | 0.00 | 0.00 | 0.00 |

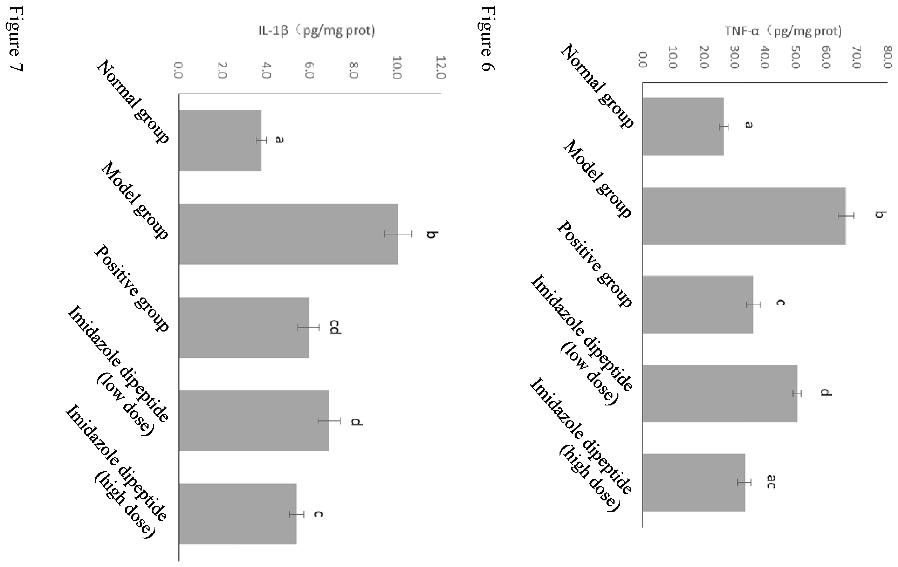

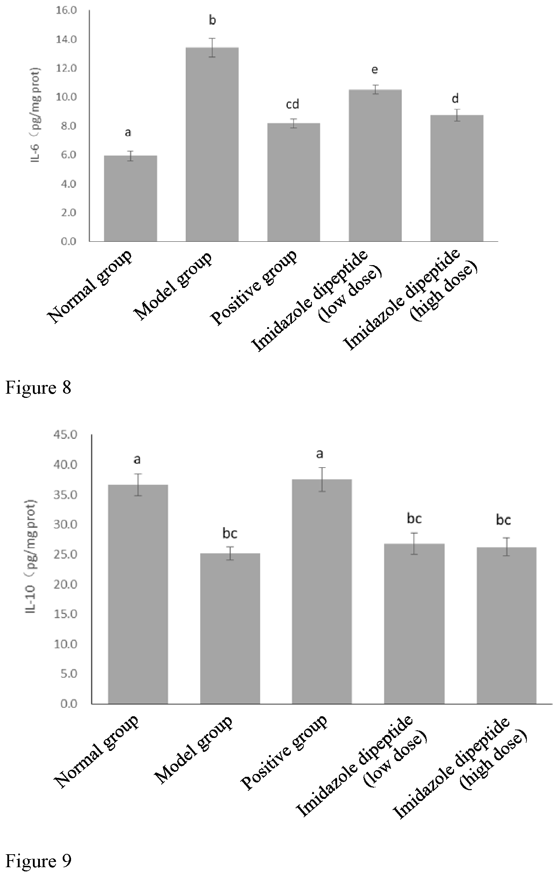

After pathological analysis of kidneys, the present disclosure further investigates the effects of hypoxanthine- and potassium oxyazinate-induced hyperuricemia on the levels of various cytokines in mouse renal tissues. As shown in FIG. 6-FIG. 9, after six weeks of modeling, compared with the normal group, the levels of pro-inflammatory factors (TNF-α, IL-1β and IL-6) in kidneys of model group mice increased significantly, while the level of anti-inflammatory factor IL-10 decreased markedly, indicating that modeling induces obvious inflammatory response in mouse kidneys. After various interventions, the levels of the three pro-inflammatory factors were significantly reduced in all intervention groups, with no significant difference in TNF-α level between the high-dose imidazole dipeptide group and the normal group.

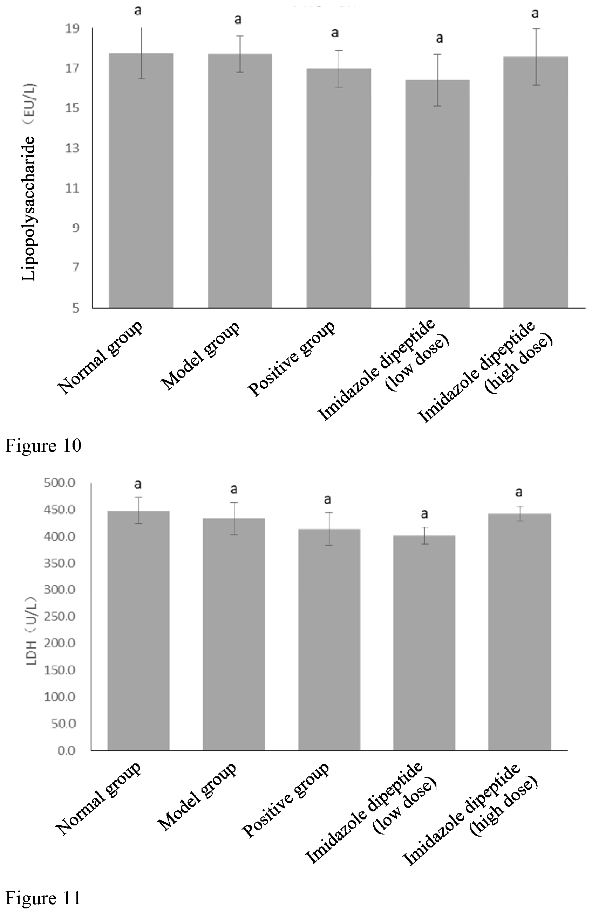

Lipopolysaccharide is a constituent of the outer cell wall of Gram-negative bacteria, a substance (i.e. glycolipid) composed of lipids and polysaccharides. Lipopolysaccharide is an endotoxin that exhibits a variety of biological activities when acting on human, animal and other biological cells. Lipopolysaccharides are toxic to the host, and endotoxins are only released after bacterial lysis upon death or artificial disruption of bacterial cells, hence the name endotoxins. It has been reported in the literature that hyperuricemia impairs intestinal permeability and triggers systemic inflammation, accompanied by elevated serum lipopolysaccharide levels. In the present experiment, no significant difference in serum lipopolysaccharide levels was found among all experimental groups (FIG. 10), indicating that renal inflammation did not progress to the extent triggering enteritis.

Lactate dehydrogenase (LDH) is a glycolytic enzyme mainly functioning to catalyze the oxidation of lactic acid into pyruvic acid and is an important enzyme in bodily energy metabolism. Lactate dehydrogenase is mainly used to assess myocardial injury and hepatocellular injury. Lesions in various tissues and organs of the body alter the intrinsic LDH of corresponding tissues and organs, thereby inducing changes in blood LDH levels. In patients with chronic glomerulonephritis, diabetic nephrosclerosis and renal malignant tumors, urinary LDH levels are generally 3 to 6 times higher than those in healthy individuals. However, urea and small molecular peptides in urine can inhibit enzyme activity. Serum LDH levels usually remain normal in chronic kidney disease patients with uremia. In this experiment, no significant difference in lactate dehydrogenase levels was observed among all experimental groups (FIG. 11).

### 6.5 Improvement effects of different interventions on oxidative stress in mice

Xanthine oxidase generates abundant superoxide anions and H₂O₂ while catalyzing the conversion of purines into uric acid, thereby elevating systemic oxidative stress in the organism. Glutathione is a major endogenous antioxidant in the body that can neutralize reactive oxygen species in vivo. Superoxide dismutase is one of the most important antioxidant enzymes in the body, which catalyzes the dismutation of superoxide anions to produce hydrogen peroxide and oxygen. Glutathione peroxidase can use reduced glutathione to catalyze hydrogen peroxide and various organic peroxides to produce water or organic alcohols. It can eliminate peroxides in living cells and play a crucial role in protecting cells against damage induced by free radicals. Lipids in cells readily react with free radicals to produce lipid peroxides. Glutathione peroxidase can reduce lipid peroxides via reduced glutathione, thereby eliminating the toxic effects induced by free radicals. Glutathione peroxidase is distributed in almost all tissues. Under certain pathological conditions, the activity of glutathione peroxidase can be significantly upregulated or downregulated. Malondialdehyde is a natural product derived from biological lipid oxidation. Lipid oxidation occurs when oxidative stress takes place in animal or plant cells, and malondialdehyde level can reflect the in vivo oxidative stress status.

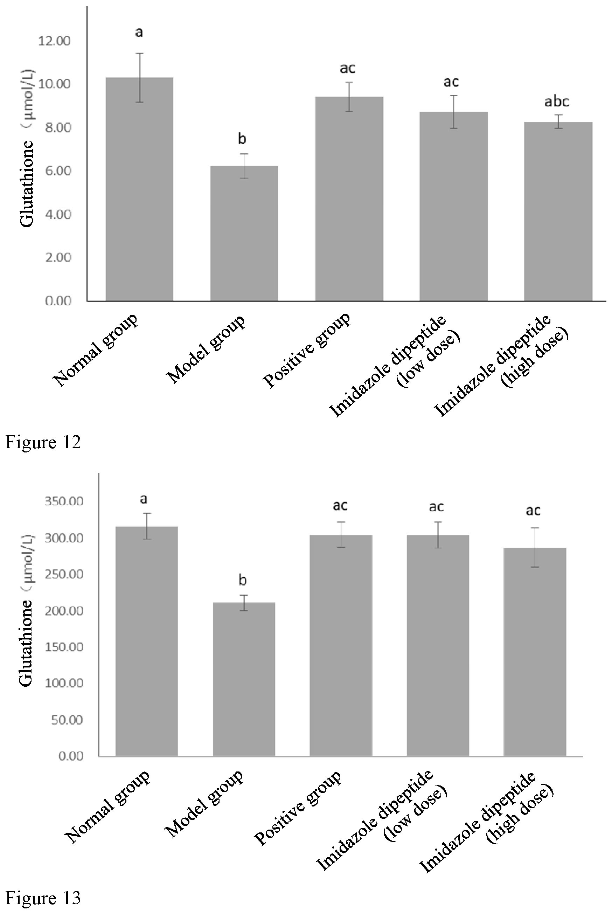

The detection results of glutathione in serum and kidney tissue homogenates (FIG. 12 and FIG. 13) showed that the glutathione concentrations in the model group were significantly lower than those in the normal group. All treatments could increase glutathione levels in serum and kidney tissue of hyperuricemic model mice and alleviate oxidative damage caused by hyperuricemia. The variation trends of glutathione concentrations in serum and kidney tissue homogenates across different experimental groups are basically consistent. Among all intervention groups, the allopurinol group and low-dose imidazole dipeptide group presented significantly higher glutathione concentrations in serum and kidney tissue homogenates than the model group, with no significant difference compared with the normal group.

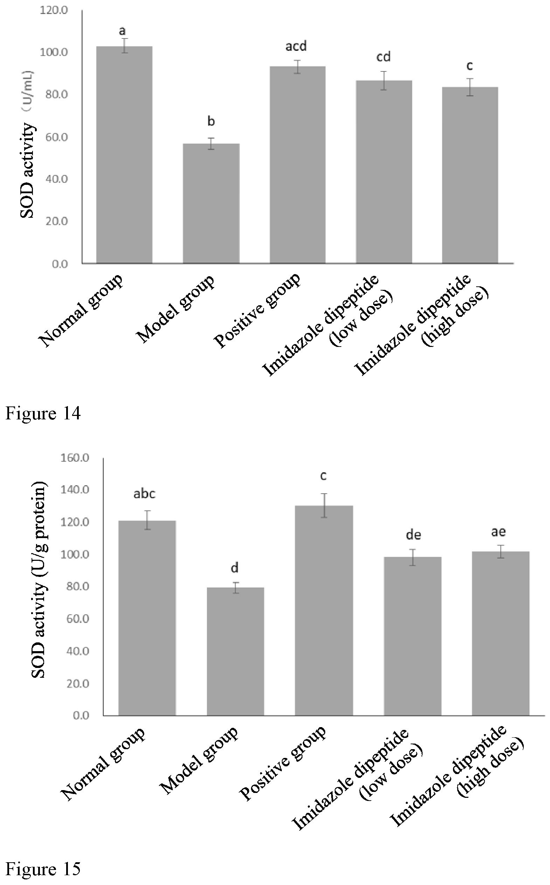

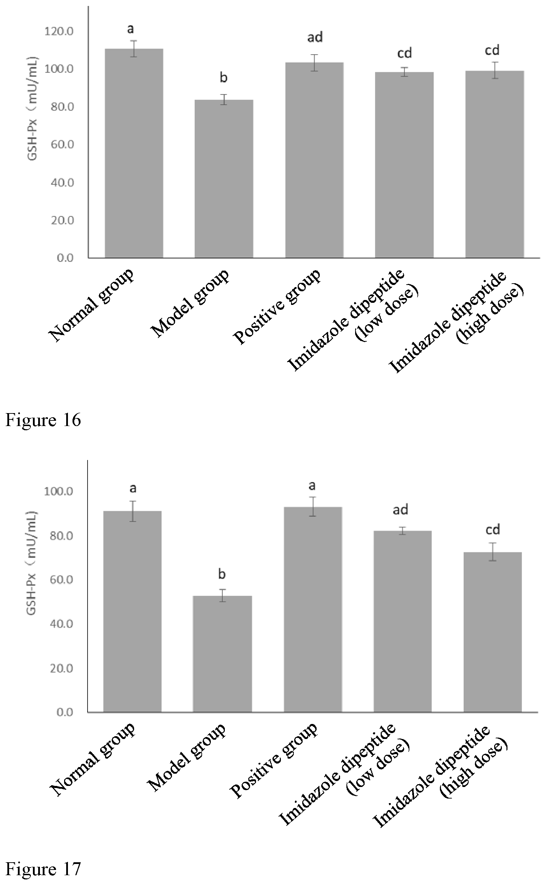

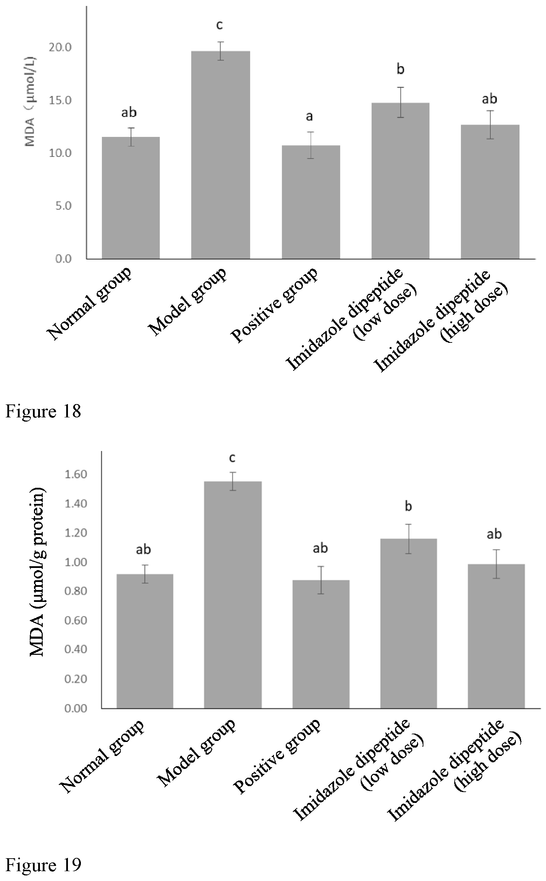

The detection results of superoxide dismutase in serum and kidney tissue homogenates (FIG. 14 and FIG. 15) showed that the superoxide dismutase activity in the model group were significantly lower than that in the normal group. All treatments could increase SOD levels in serum and kidney tissue of hyperuricemic model mice and alleviate oxidative damage caused by hyperuricemia. The variation trends of SOD activity in serum and kidney tissue homogenates across different experimental groups are basically consistent. Among all intervention groups, the allopurinol group and high-dose imidazole dipeptide group presented significantly higher SOD activities in serum and kidney tissue homogenates than the model group. The detection results of glutathione peroxidase in serum and kidney tissue homogenates (FIG. 16 and FIG. 17) showed that the glutathione peroxidase activities in the model group were significantly lower than those in the normal group. All treatments could increase glutathione peroxidase levels in serum and kidney tissue of hyperuricemic model mice and alleviate oxidative damage caused by hyperuricemia. The variation trends of glutathione peroxidase activities in serum and kidney tissue homogenates across different experimental groups are basically consistent. Among all intervention groups, the allopurinol group, high- and low-dose imidazole dipeptide groups presented significantly higher glutathione peroxidase activities in serum and kidney tissue homogenates than the model group. The detection results of malondialdehyde in serum and kidney tissue homogenates (FIG. 18 and FIG. 19) showed that the malondialdehyde concentrations in the model group were significantly higher than those in the normal group. All treatments could significantly reduce malondialdehyde levels in serum and kidney tissue of hyperuricemic model mice. The malondialdehyde levels in all intervention groups exhibited no significant difference compared with the normal group, indicating that all treatments could alleviate oxidative damage caused by hyperuricemia.

The above experiments show that the imidazole dipeptide composition of the present disclosure has renal protective effects.

## Claims

1. An imidazole dipeptide composition with a renal protective effect, comprising anserine and carnosine, wherein based on 100% of the total weight of the anserine and the carnosine, the content of anserine is 10% to 99% and the content of carnosine is 1% to 90%.

2. The imidazole dipeptide composition according to claim 1, wherein based on 100% of the total weight of the anserine and the carnosine, the content of anserine is 91% and the content of carnosine is 9%.

3. The imidazole dipeptide composition according to claim 1 or 2, consisting of anserine and carnosine.

4. The imidazole dipeptide composition according to claim 1 or 2, further comprising other substances with a renal protective effect.

5. The imidazole dipeptide composition according to claim 4, wherein the other substances with a renal protective effect include histidine and/or a histidine derivative; preferably, wherein the histidine derivative includes, but is not limited to: an oligopeptide containing a histidine residue, more preferably a dipeptide or tripeptide containing a histidine residue;
preferably, wherein the histidine and the histidine derivative are derived from animals and/or plants, such as from chicken, fish or other animal muscles.

6. Use of the imidazole dipeptide composition according to any one of claims 1-5 in the manufacturing of a composition with a renal protective effect.

7. The use according to claim 6, wherein the composition is for use in manufacturing a medicament.

8. The use according to claim 7, wherein the medicament contains an effective amount of the imidazole dipeptide composition.

9. The use according to claim 7 or 8, wherein the dosage of the imidazole dipeptide composition in the medicament is 1 mg/day to 200 mg/day, preferably 1 mg/day to 100 mg/day, and more preferably 5 mg/day to 50 mg/day.

10. The use according to any one of claims 7-9, wherein the medicament further contains an excipient, preferably, wherein the excipient includes one or more of sorbitol, maltodextrin, and magnesium stearate.

11. The use according to any one of claims 7-10, wherein the medicament is in the form of a tablet, a capsule, powder, or a granule.

12. The use according to claim 6, wherein the composition is for use in manufacturing a food product.

13. The use according to claim 12, wherein the amount of the imidazole dipeptide composition in the food product is 1 mg/day to 200 mg/day, preferably 1 mg/day to 100 mg/day, and more preferably 5 mg/day to 50 mg/day.
